Europäisches Patentamt

European Patent Office

Office européen des brevets

(19)

(11) Publication number : **0 508 834 A1**

## (12) EUROPEAN PATENT APPLICATION

(21) Application number : 92303289.0

(22) Date of filing : 13.04.92

(51) Int. Cl.⁵ : **G01N 33/569**

(30) Priority : **11.04.91 US 683976**

(43) Date of publication of application :
**14.10.92 Bulletin 92/42**

(84) Designated Contracting States :
**AT BE CH DE DK ES FR GB GR IT LI LU MC NL
PT SE**

(71) Applicant : **CEDARS-SINAI MEDICAL CENTER
8700 Beverly Boulevard
Los Angeles, California 90048-1869 (US)**

(72) Inventor : **Brunell, Philip Alfred
200 North Swall Drive, No. 401
Beverley Hills, California 90211 (US)**
Inventor : **Srugo, Isaac
827 Levering Avenue, No. 201
Los Angeles, California 90024-276 (US)**

(74) Representative : **Sexton, Jane Helen et al
J.A. Kemp & Co. 14 South Square Gray's Inn
London WC1R 5LX (GB)**

(54) **Immunoassay for the detection of HIV specific IgM.**

(57) A method of immunoassay for detecting antibodies to human immunodeficiency virus (HIV). The assay detects HIV specific IgM antibody thereby distinguishing those infants who are infected with HIV from those who have passively acquired maternal HIV specific IgG antibodies. Additionally, the assay can be used to detect individuals infected with HIV who have not yet mounted an IgG immune response.

EP 0 508 834 A1

Field Of The Invention

This invention relates to immunodiagnostic methods for detecting antibody to human immunodeficiency virus (HIV). More particularly, the invention relates to an enzyme-linked immunosorbent assay (ELISA) for the detection of HIV specific immunoglobulin M (IgM). One embodiment of the present invention provides a specific test for the detection of those newborns who have actually contracted HIV infection in utero from among all children born to an HIV seropositive mother. Specifically, the invention allows the differentiation of antibodies produced by an infant's autogenous anti-HIV antibody response from those maternal anti-HIV antibodies acquired passively through prepartum maternal/fetal circulation.

Background Of The Invention

Since its initial recognition in 1981, acquired immunodeficiency syndrome (AIDS) has become a global pandemic. The World Health Organization estimates that 5-10 million people worldwide are infected with AIDS. Statistics from the Center for Disease Control in Atlanta suggest that before 1991 is over, there will be 12.5 million HIV-infected Americans, and 10,000 to 20,000 of these may be infected infants and children. (Oleske, J.: National History of HIV Infection. Report of Surgeon General's Workshop on Children With HIV Infection and Their Families, DHHS Pub. No. HRS-D-MC 87-1, Washington, D.C.: Supt. of Docs., U.S. Gov't Print. Off., 1987; 24-25. The Public Health Service places the number between 800,000 and 1.2 million. CDC: Estimate of HIV Prevalence and Projected AIDS Cases: Summary of a Workshop, Oct 31-Nov 1, 1989; MMWR 39:7;110-119.

The etiologic agent of AIDS is the human immunodeficiency virus, a member of the -Family Retroviridae, and known as HIV, HTLV-III or ARV. Current evidence shows that HIV disease is transmitted primarily by contact with, or exposure to, HIV-infected body fluids. As of February, 1990, 2116 cases of Pediatric Aids had been reported to the CDC. Eighty percent of these cases were attributable to maternal transmission of the disease during the perinatal period. (U.S. Congress: Hearing before the Select Committee on Children, Youth and Families, Beyond the Stereotypes: Women, Addiction, and Perinatal Substance Abuse; April 19, 1990, p. 7.)

Children are members of the fastest growing group of reported AIDS patients. Scientific American, Oct., 1988. It was not until 1982 that pediatric AIDS was discussed in the scientific literature, yet it is already the ninth leading cause of death among children ages 1 to 4 years of age. It is estimated that in 1991 the total number of HIV-infected children will total between 10,000 and 20,000. This can be translated to mean one of every ten pediatric hospital beds will be occupied by a child with AIDS at a total cost of over $1 billion. Secretary's Work Group on Pediatric HIV Infection and Disease: Final Report, DHHS Pub. No. NIH 89-3063, Washington, D.C.; Supt. of Docs. U.S. Gov't Print. Off. 1988, 12-13. Present information suggests that 25-35% of babies born to infected mothers will contract HIV-disease. Perinatal transmission can occur: (1) prenatally, by transplacental passage of the virus in utero; (2) during delivery, by exposure to infected maternal blood and vaginal fluids; and (3) during the postpartum period, probably by the ingestion of breast milk containing the virus. Aids and Other Manifestations of the HIV-Infection, Noyes Publications, Park Ridge, New Jersey (1987), Chap. III, The Epidemiology of Pediatric HIV Infections, pp. 42-43. [HIV infection progressively impairs a patient's immune system. One result is an individual more susceptible to opportunistic infections. Adults infected with HIV are likely to have lymphopenia and severe cell-mediated immunodeficiency, with a dramatic deficiency of helper T-cells, as well as hyper-gammaglobulinemia and a diminished capacity to produce antibody After primary or booster immunization. J. of Ped., 110:563-566 (1987). Opportunistic infections or malignancies characteristic of adult HIV disease include Pneumocystis carni pneumonia, cytomegalovirus retinitis, and Kaposi's sarcoma. While HIV infection produces similar opportunistic infections and immunological abnormalities in infants, congenitally acquired HIV infection will more profoundly affect the infant's central nervous system resulting in alterations of growth and development.

For an unknown period of time immediately after infection with HIV, an infected individual may experience an antibody negative state, i.e., seronegativity. During this time HIV is detectable only by viral culture studies. Later, an infected individual will often manifest a brief mononucleosis-like illness with fever, malaise and skin rash. This symptomology usually signifies seroconversion, i.e., the time anti-HIV antibodies can first be detected. Seroconversion is thought to occur between two weeks and three months following initial infection. The Merck Manual, 15th Ed., Merck & Co., Inc., Rahway, N.J., 1987.

Detection of perinatal transmission of AIDS in newborns is complicated by the lack of a reliable diagnostic test. Scientific American, Oct., 1988 at 80. Since immunoglobulin G (IgG) antibody is quantitatively transferred from mother to fetus across the placenta, infants born to HIV-infected mothers will have passively acquired, maternally-derived anti-HIV antibodies circulating in their blood regardless of whether or not they have in fact been infected with the virus. Thus, HIV immunodiagnostic methods based on detection of anti-HIV IgG will produce a false positive result in these infants. This diagnostic nonspecificity may continue beyond the infant's first birthday because maternal anti-HIV IgG antibodies persist in the child's blood for up to 14 months.

In contrast, as a response to intrauterine infection with HIV, the fetus synthesizes Its own anti-HIV antibodies. While all classes of antibody are eventually produced, anti-HIV IgM is the initial fetal response. Since IgM antibody cannot cross the placenta, its presence in cord serum is pathognomonic of fetal intra-uterine HIV infection. Because present diagnostic methods do not distinguish IgM antibodies from IgG antibodies, they likewise fail to distinguish active fetal disease from the mere transient seropositivity acquired passively from an infected mother. This distinction can be critical, however, given the high mortality rate associated with pediatric HIV disease. An infected child is very likely to die or be severely ill by two years of age. Early confirmation of HIV infection is therefore essential so that appropriate instructions for care can be given and regular medical follow-up can be instituted. In addition, with the development of anti-viral drugs, early diagnosis and recognition is imperative for early treatment.

Protection of the infant who has escaped in utero infection is also an important goal of the early differentiation between apparent and actual infant seroconversion. At least one case has been reported where an infant acquired HIV infection postnatally from its mother, who had herself acquired the disease from a post delivery transfusion of HIV infected blood. Zeigler, J.B., et al.: Postnatal Transmission of AIDS-Associated Retrovirus from Mother to Infant, Lancet 1985; 1: 896-897. Realization that an infant's apparent seroconversion merely represents the transient presence of material antibodies will allow steps to be taken to protect this infant without the necessity of preventing breast feeding and other mother-child contact for those infants who are in fact already infected.

As previously indicated, the diagnosis of HIV infection relies upon laboratory confirmation of the presence of either HIV or anti-HIV antibodies in a given patient specimen. Virus isolation techniques can detect the presence of HIV in serum but are impractical for early screening since the process is cumbersome and expensive. Cultivation requires inoculation of virus-containing materials into living cell cultures, which requires costly reagents and long culture time (generally about four weeks).

Antigen detection systems have been developed which might be useful in detecting HIV infection. These methods permit direct detection of the viral antigen present in a patient's serum, thus avoiding the delay of waiting for cultivation of the virus in a cell culture system. To date however, these tests have been insensitive, perhaps because the antigen is present in vivo in low concentrations or because of interfering or non-specific materials.

Other diagnostic tests for HIV infection look for a specific patient response such as the production of specific antibodies. One such test searches for the presence of patient antibodies capable of blocking HIV infectivity. As discussed above, current systems are not capable of distinguishing the class of antibody which neutralizes HIV. Thus they are useless in the newborn population as well as in very early adult HIV disease.

The enzyme linked immunosorbent assay (ELISA) is currently the standard method for sensitive screening of antibodies to the AIDS virus. This test lacks specificity, however, and a positive result must be confirmed with Western blot analysis or radio-immunoprecipitation assay, especially if the appropriate clinical picture is absent. (Manual of Clinical Immunology, 3rd Ed., Rose, Friedman & Fahey, American Society for Microbiology 1986.) Another disadvantage of the standard ELISA systems is that they are directed against IgG antibody and cannot specifically detect IgM antibody.

The procedure for the indirect ELISA systems is essentially as follows:

1. A solid phase is sensitized by passive absorption with the relevant antigen.
2. Test serum is added; any antigen-specific antibody in the serum reacts with the immobilized antigen.
3. Enzyme-labeled anti-human immunoglobulin is added; this conjugate reacts with any antibody attached to the solid phase-immobilized antigen.
4. Enzyme substrate is added; the color intensity of the solution is estimated visually or photometrically.

The standard ELISA method is generally useful for the detection of total antibody or IgG antibody. The method is not as effective for IgM antibody assay because the antigen is affixed directly to a solid phase, and IgG antibody in the test serum may attach to the antigen creating two problems. First, false negative results may occur if the IgG antibody is far in excess of the IgM since all of the antigen combining sites may be bound to IgG and the IgM will not be detected. Second, rheumatoid factor (RF) may cause false positive results. RF is an IgM antibody directed nonspecificently against all species of IgG antibody. Thus, during the ELISA, any RF present combines with HIV specific IgG. When the remaining reagents are added a positive test results occurs, even if no anti-HIV IgM is present.

## Summary Of The Invention

The foregoing discussion describes the techniques currently available to detect HIV antibody in serum.

None of these techniques is of adequate sensitivity and specificity to distinguish between anti-HIV antibody of the IgG and IgM classes. A special antibody capture ELISA method has been developed to detect IgM anti-HIV antibodies. One embodiment of this invention is an assay that allows the identification of infants who have been actually infected with the human immunodeficiency-virus in utero and distinguish these infants from infants who have merely acquired transient maternal antibodies passively through the maternal/fetal circulation. Since IgM antibody is not transported across the placenta, its presence in the cord sera rules out the possibility of passive acquisition of maternal antibody and is thus pathognomonic of true fetal infection.

The availability of this IgM assay to identify infected infants would permit treatment of HIV with antiviral agents soon after the onset of the disease. It would also identify those exposed infants who were not yet infected and who might benefit from preventive efforts, e.g., passive immunization, which have been successful in Hepatitis B infections. Further, it would determine which infants require special precautions to prevent spread of the virus.

Although the method described may be used to detect anti-HIV IgM antibody in infants suspected of in utero infection, it is not intended to be restricted to this use. The method may also be used, for example, to detect anti-HIV IgM antibody in adults who are either incapable of mounting an IgG response, or who are so recently infected that they have not yet done so.

Detailed Description Of The Invention

The method of the invention allows detection of IgM antibody directed against HIV (anti-HIV IgM). Specifically, an anti-HIV IgM ELISA is described which is capable of detecting infected infant patients prior to their development of symptoms of infection. Likewise, infected adult patients and blood donors can be detected prior to the time they become seropositive by current anti-HIV IgG ELISA.

Infants born to HIV infected mothers acquire anti-HIV IgG transplacentally and are classified as PO, an intermediate category, until their status can be definitely determined. IgM is not transported across the placenta. Therefore, the presence of anti-HIV IgM in cord sera indicates that it must have resulted from fetal rather than maternal synthesis. A screening test for anti-HIV IgM in newborns would thus be invaluable for early identification of infected infants.

Following infection with HIV, anti-HIV IgM is the initial antibody response; it precedes the synthesis of anti-HIV IgG. It follows that a method of detecting anti-HIV IgM can also be utilized to identify infected patients and donors earlier in disease progression than is possible by conventional anti-HIV IgG ELISA.

The size of the antigen required for a sensitive assay is critical. It has been confirmed previously that an IgM HIV ELISA using a detergent lysed antigen was insensitive. An antibody capture technique is therefore employed to circumvent problems associated with the conventional ELISA, such as those produced by the presence of rheumatoid factor or excess IgG. The result is an anti-HIV IgM ELISA capable of detecting infected adult patients and infants before developing symptoms of infection and infected adult patients and donors prior to the time they become seropositive by currently available methods.

The advantage of the present invention can readily be seen in the following descriptions of the preferred embodiment. Of course, these examples are merely illustrative and are not intended to limit the claimed invention.

Example 1

Passage of various strains of HIV-1 in H-9 cells is well described in the literature. Cultures are harvested when extensive syncytial formation is observed. The cell pellet of infected H-9 cells is washed twice with phosphate buffered saline (PBS), pH 7.4, and aliquots are used to prepare antigen by freezing a preparation of infected H-9 cells at -70°C and slow thawing for three hours at 4°C. This sample is then treated with a final concentration of 0.5% Triton X-100 for 5 minutes at room temperature to destroy infectivity.

Flat U-shaped polyvinyl chloride microliter plates (Nunc Immune Plate-Maxisorp Intermed, Denmark) are coated with 0.1 ml of a 1:200 caprine antihuman $\mu$ antibody (IgM) (Tago, Inc., Burlingame, CA) in carbonate buffer, pH 9.6. Plates are stored at 4°C for a minimum of 72 hours before use. Coating fluid is removed by washing five times with phosphate buffered saline containing 0.5% tween 20 (PBS-T) including three soaks of 1.5 minute duration each; all subsequent washings are done in this manner.

Serial dilutions of all reagents are tested in various combinations to achieve the clearest separation of anti-HIV IgM seropositives and seronegatives. All reagents are added in 0.1 ml volumes. A 1:100 dilution of each test serum sample is prepared in 1% heat inactivated fetal calf serum containing 10% globulin poor normal goat serum in PBS-T. Incubation of the sera is performed at 37°C in a moist chamber of three hours. A 1:10 dilution of antigen or appropriate controls in 15% globulin poor normal goat serum in PBS-T is then added to each of two "viral" or "control" wells, respectively. Next, incubation is performed at 18°C in a moist chamber overnight. Incubation of the antigen at 37°C for shorter periods of time yield lower values. Antisera are incubated for 4 hours at 37°C. A 1:1000 dilution of goat anti-rabbit serum conjugated to horseradish peroxidase (Tago, Burlingame, CA) is then incubated for 2 hours at 37°C. Finally, the substrate (0-phenylenediamine 2HCl), diluted in citrate phosphate buffer containing 0.02% hydrogen peroxidase is added. After incubation for 30 minutes at room temperature, the enzymatic reaction is stopped by the addition of 1N $H_2SO_4$ and the color reaction is read spectrophotometrically with an enzyme immunoassay DuPont reader (model 901, DuPont, Inc., N. Billerica, MA) at 490 nm.

The result is expressed as the delta optical density (DOD), i.e., the mean of the optical density of two antigen wells minus the mean of the two control wells. Aliquots of a known negative serum sample, as well as low and high-positive serum samples, are included in each run. Assays in which there is a significant variation from the mean value found in previous assays of either serum are rejected.

Example 2

Utilizing the method described above in Example Ex. 1, nineteen infants born to HIV infected mothers were tested. All but two were tested blindly. Twelve were uninfected and seven were infected. Blood samples from five of the latter were available during the first six weeks of life before they developed clinical symptoms of infection (PO). The age range of all the infants was one day to six months. The determination of infection was done by a positive blood culture for HIV and/or by developing clinical symptoms of infection. (Five infants had positive blood cultures and two had clinical symptoms of infection. One of them had both.)

A group of 22 cord sera obtained from normal newborns were-tested for anti-HIV IgM to define a seronegative range. The mean and standard deviation for 22 cord sera were, respectively, 0.040 and 0.036. A DOD value of 0.068, i.e., three standard deviations plus the mean, included all 22 cord sera was defined as the upper limit of the seronegative values.

All seven infected infants were positive for IgM-HIV (range 0.107-454). Six who were tested within the first six weeks of life were positive, only one of whom had clinical signs of infection. All three cord sera tested were HIV-IgM positive (see Table 1). Only one of the 12 uninfected infants was positive (0.161) in our anti-HIV IgM assay.

## TABLE 1

### HIV IgM Values Related to Age and Clinical Status in Seven Infected Infants

| Patient | Age Tested | CDC Classification (1) | HIV IgM |
|---------|------------|------------------------|---------|
| 1 | 2 WEEKS | P-2A | (+) 0.106 |
| 2 | 6 WEEKS | P-0 | (+) 0.422 |
| 3 | 4 WEEKS | P-0 | (+) 0.233 |
| 4 | CORD SERA | P-0 | (+) 0.125 |
|   | 3 WEEKS | P-2B | (+) 0.215 |
| 5 | CORD SERA | P-0 | (+) 0.113 |
|   | 4 WEEKS | P-1 | (+) 0.191 |
| 6 | CORD SERA | P-0 | (+) 0.158 |
|   | 5 WEEKS | P-1 | (+) 0.120 |
| 7 | 6 MONTHS | P-2A | (+) 0.454 |

The upper limit of the seronagative was 0.068.

5

Example 3

Seven plasma donors who had developed anti-HIV IgG during a period when they were bled at two to seven day intervals were tested blindly. Stored plasma samples, at least some of which antedated the seroconversion, were used for anti-HIV IgM testing. Two to six samples from each donor were tested.

All seven donors were positive for anti-HIV IgM. Twenty-seven of 29 adult sera tested had demonstrable anti-HIV IgM. The only serum specimens which were seronegative were the initial samples from two donors. Nine of 29 samples were negative for anti-HIV IgG, 13 of them were positive and the rest had intermediate values.

**Claims**

1. A method of detecting immunoglobulin M (IgM) antibodies to human immunodeficiency virus (HIV) in a body fluid comprising:-
   a) attaching anti-human IgM antibody to a solid support;
   b) adding body fluid to the solid phase-antibody complex, incubating and washing;
   c) adding HIV antigen, incubating and washing;
   d) adding anti-HIV antibody, incubating and washing;
   e) adding anti-(anti-HIV antibody)-antibody conjugated to an indicator system and incubating; and
   f) detecting the presence of the bound or unbound indicator.

2. A method of detecting immunoglobulin M (IgM) antibodies to human immunodeficiency virus (HIV) In human body fluid comprising:-
   a) attaching anti-human IgM antibody to a solid support;
   b) "postcoating" the solid phase-antibody complex with bovine serum albumin;
   c) adding body fluid, incubating and washing;
   d) adding HIV antigen, incubating and washing;
   e) adding anti-HIV antibody, incubating and washing;
   f) subsequently adding anti-(anti-HIV antibody) antibody conjugated to an indicator system and incubating; and
   g) detecting the presence of the bound or unbound indicator.

3. A method according to claim 1 or 2 wherein the body fluid is serum, saliva, sputum, sperm or tissue extract.

4. A method according to claim 1 or 2 wherein the body fluid is umbilical cord blood or serum, or blood or serum from an infant.

5. A method according to any one of the preceding claims wherein the indicator system employs an enzyme or a radiolabel.

6. A method according to any one of claims 1 to 4 wherein detecting the presence of the indicator comprises addition of an enzyme substrate that yields a visible color reaction.

7. A method according to any one of claims 1 to 4 wherein detecting the presence of the indicator comprises scintillation scanning for a radiolabeled antibody.

8. A method of detecting immunoglobulin M (IgM) antibodies to human immunodeficiency virus (HIV) in human serum comprising:-
   a) coating microliter plates with dilutions of anti-human IgM antibody in carbonate buffer, pH 9.6;
   b) incubating the plates at about 4°C for at least 10 hours;
   c) decanting and washing the solid phase;
   d) "post coating" the plates with about 1% bovine serum albumin applied for about one hour at about 37°C and then washing;
   e) adding diluted serum and incubating for about one hour at about 37°C;
   f) adding HIV antigen, incubating and then washing;
   g) adding an antibody against HIV, incubating and washing;
   h) adding antibody against the antibody species in step g conjugated with alkaline phosphatase and incubating;

i) adding a color generating substrate as an indicator and stopping the reaction of the substrate after about 45 minutes, preferably by adding sodium hydroxide; and
j) using an ELISA reader to read the color generated.

9. A method according to claim 8 wherein the human serum is obtained from umbilical cord blood or from an infant.

10. A method according to any one of claims 1 to 9 wherein the antibodies are polyclonal.

11. A claim according to any one of claims 1 to 9 wherein the antibodies are monoclonal.

12. A claim according to claim 8 wherein the indicator system employs an enzyme or a radiolabel.

**European Patent Office**

## EUROPEAN SEARCH REPORT

Application Number

EP 92 30 3289

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | WO-A-8 807 680 (THE PUBLIC HEALTH LABORATORY SERVICE BOARD) <br> * page 1 - page 10 * <br> * page 21, line 10 - line 18 * <br> --- | 1-3,5-8, 10-12 | G01N33/569 |
| X | EP-A-0 292 810 (BEHRINGWERKE AG) <br><br> * page 2, line 20 - page 4, line 8 * <br> --- | 1,2,5,6, 11-12 | |
| A | WO-A-8 910 980 (BIO-RESEARCH LABORATORIES, INC.) <br><br> * page 4, paragraph 3 - page 5, paragraph 3 * <br> * page 11, line 6 - line 23 * <br> --- | 1,3,5,6, 8 | |
| A | EP-A-0 265 851 (BEHRINGWERKE AG) <br><br> * page 2, line 33 - line 40 * <br> * page 3, line 23 - page 4, line 34 * <br> * page 5, line 2 - line 12 * <br> --- | 1-3-5,6, 8,10,11 | |
| A | WO-A-8 801 305 (THE UNITED STATES OF AMERICA) <br> * page 3, line 6 - line 10 * <br> * page 9, line 22 - page 10, line 14 * <br><br> ----- | 4,9 | TECHNICAL FIELDS SEARCHED (Int. Cl.5 ) <br><br> G01N |

The present search report has been drawn up for all claims

| Place of search | Date of completion of the search | Examiner |
|---|---|---|
| BERLIN | 04 JUNE 1992 | DE KOK A.J. |